# EUROPEAN PATENT APPLICATION

(11) **EP 3 851 164 A1**
(43) Date of publication of application: **21.07.2021**
(21) Application number: 19878433.2
(22) Date of filing: 18.10.2019
(51) Int. Cl.: A61N 7/02, A61B 8/14, H04R 17/00

(54) **ULTRASOUND EMISSION DEVICE AND ULTRASOUND APPARATUS**

(30) Priority: 30.10.2018 JP 2018203766
(71) Applicant: Kyocera Corporation, Kyoto-shi, Kyoto 612-8501 (JP)
(72) Inventor: SATO, Izuru, Kyoto-shi, Kyoto 612-8501 (JP); TAKAHASHI, Tooru, Kyoto-shi, Kyoto 612-8501 (JP); NINOMIYA, Hiroshi, Kyoto-shi, Kyoto 612-8501 (JP); MIYAZATO, Kentarou, Kyoto-shi, Kyoto 612-8501 (JP)
(74) Representative: Viering, Jentschura & Partner mbB Patent- und Rechtsanwälte
(86) International application number: PCT/JP2019/041149
(87) International publication number: WO 2020/090525

(57) **Abstract**

An ultrasound emission device includes a generation part which generates an ultrasonic wave emitted to an object side, and a bag in which a liquid is sealed so that the liquid is located on the object side relative to the generation part. The generation part includes a piezoelectric element which generates vibration generating the ultrasonic wave. The piezoelectric element includes a first surface facing the object side and a second surface facing an opposite side to the object side. The piezoelectric element includes, between the first surface and the second surface, a piezoelectric layer which extends along the first surface and the second surface, and a pair of electrodes which are superposed on two surfaces of the piezoelectric layer. The piezoelectric element flexurally deforms so that the first surface and the second surface flex to a same side as each other. The first surface is exposed to the inside of the bag so as to contact the liquid. The second surface is isolated from the liquid and is in contact with a gas.

## Description

### Technical Field

The present disclosure relates to an ultrasound emission device emitting ultrasonic waves toward a human body or other object and an ultrasound apparatus having the ultrasound emission device.

### Background Art

Known in the art is an ultrasound apparatus emitting ultrasonic waves toward a human body or other object (for example Patent Literature 1) . Such an ultrasound apparatus is for example utilized as an ultrasound therapeutic apparatus for treatment by emitting ultrasonic waves to an affected area etc. or an ultrasound diagnosis apparatus for acquiring a cross-sectional two-dimensional image of an affected area or the like. As an ultrasound therapeutic apparatus, for example, there is known an apparatus utilized for HIFU (high intensity focused ultrasound) treatment. Patent Literature 1 discloses an ultrasound therapeutic apparatus having a generation part which generates ultrasonic waves and emits the same toward a human body and a water bag which is interposed between the generation part and the human body. The water bag for example contributes to dampening of a sudden change of acoustic impedance between the generation part and the human body.

### Citation List

### Patent Literature

Patent Literature 1: Japanese Patent Application Laid-Open No. H01-139052

### Summary of Invention

An ultrasound emission device according to one aspect of the present disclosure includes a generation part which generates an ultrasonic wave emitted to an object side, and a bag in which a liquid is sealed so that the liquid is located on the object side relative to the generation part. The generation part includes a piezoelectric element which generates vibration generating the ultrasonic wave. The piezoelectric element includes a first surface facing the object side and a second surface facing an opposite side to the object side. Further, the piezoelectric element includes, between the first surface and the second surface, a piezoelectric layer which extends along the first surface and the second surface and a pair of electrodes which are superposed on two surfaces of the piezoelectric layer. The piezoelectric element flexurally deforms so that the first surface and the second surface flex to a same side as each other by application of voltage to the pair of electrodes. The first surface is exposed to the inside of the bag so as to contact the liquid. The second surface is isolated from the liquid and is in contact with a gas.

An ultrasound apparatus according to one aspect of the present disclosure includes the ultrasound emission device explained above and a driving control part which supplies an AC voltage having a frequency in a frequency band of ultrasound to the pair of electrodes.

### Brief Description of Drawings

FIG. 1 is a schematic view showing a schematic configuration of an ultrasound apparatus according to an embodiment.
FIG. 2 is a schematic view showing a schematic configuration of an ultrasonic wave generation part in the ultrasound apparatus in FIG. 1.
FIG. 3 is a plan view of a flat plate element in the ultrasonic wave generation part in FIG. 2.
FIG. 4 is a cross-sectional view taken along the IV-IV line in FIG. 3.
FIG. 5 is an enlarged view of a portion in FIG. 4.
FIG. 6A and FIG. 6B are schematic views for explaining the effects of the ultrasonic wave generation part in FIG. 2.
FIG. 7A and FIG. 7B are cross-sectional views showing a modification and another modification.

### Description of Embodiment

Below, an embodiment according to the present disclosure will be explained with reference to the drawings. The following drawings are schematic ones. Accordingly, sometimes details will be omitted. Further, the size ratios etc. will not always coincide with the actual ones. Further, the size ratios among a plurality of drawings will not always coincide with each other either.

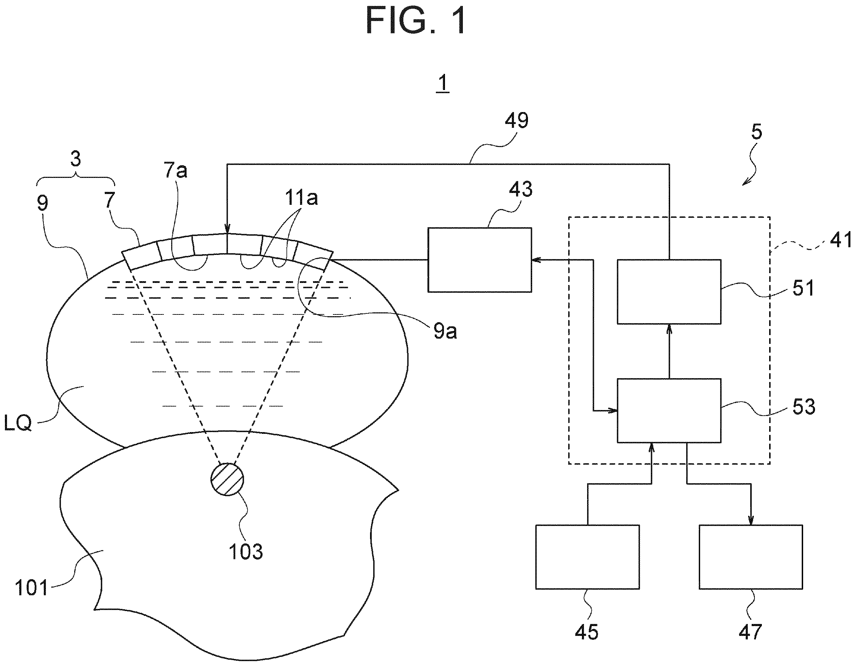

FIG. 1 is a schematic view showing a schematic configuration of an ultrasound apparatus 1 according to an embodiment.

The ultrasound apparatus 1 is for example one utilized for HIFU treatment. For example, the ultrasound apparatus 1 focuses the ultrasonic waves at an affected area 103 (or calculus or other foreign substance, same is true for the following explanation) in a patient 101. Heat etc. generated by this causes the affected area 103 to change in nature. The ultrasound apparatus 1 may be configured to treat any part in the human body as the treated object. Further, the ultrasound apparatus 1 may be configured to treat any disease as the treated object as well. In other words, the frequency and strength of the ultrasonic waves emitted by the ultrasound apparatus 1 and the dimensions etc. of the parts in the ultrasound apparatus 1 may be suitably set. Note that, ultrasonic waves are generally considered acoustic waves of 20 kHz or more. There is no particular general upper limit on the frequency of the ultrasonic waves. However, for example, the upper limit may be made 5 GHz.

The ultrasound apparatus 1 has an ultrasound emission device 3 which is arranged adjacent to the patient 101 and directly responsible for emission of ultrasonic waves (below, sometimes simply referred to as the "emission device 3") and an apparatus body 5 supplying power to the emission device 3 etc.

### (Ultrasound emission device)

The emission device 3 has a generation part 7 generating ultrasonic waves and a bag 9 interposed between the generation part 7 and the patient 101. The generation part 7 for example emits ultrasonic waves from an emission surface 7a facing the patient 101 side. The emission surface 7a is substantially configured as a recessed shape. The recessed shape is for example substantially shaped as a partially cut away sphere. Accordingly, the ultrasonic waves emitted from the emission surface 7a are focused at the center of the sphere (from another viewpoint, affected area 103) . The bag 9 has a liquid LQ sealed in it at least when the ultrasound apparatus 1 is used. The liquid LQ for example contributes to dampening of any rapid change of the acoustic impedance between the emission surface 7a and the body surface of the patient 101.

The liquid LQ is for example water. Further, for example, the liquid LQ may be one including water and a suitable additive as well. The additive may be for example one for adjusting the acoustic impedance. The acoustic impedance of the liquid LQ may be made for example 1×10⁶ kg/(m²•s) to 2×10⁶ kg/(m²•s) or 1.3×10⁶ kg/(m²•s) to 1.7×10⁶ kg/(m²•s). If illustrating the acoustic impedances of various substances for reference, water is about 1.5×10⁶ kg/(m²•s), air is about 0, fat is about 1.4×10⁶ kg/(m²•s), and muscle is about 1.7×10⁶ kg/(m²•s).

### (Generation Part)

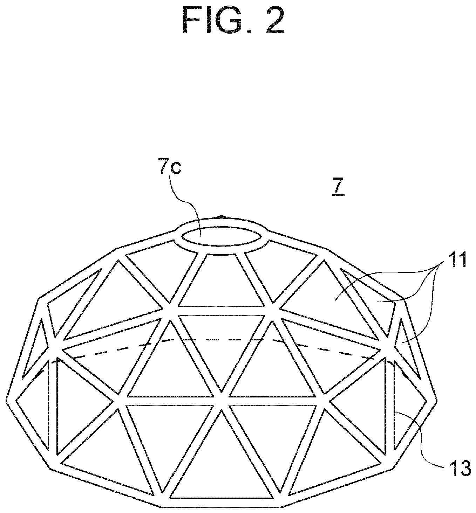

FIG. 2 is a schematic view for explaining the configuration of principal parts in the generation part 7. In FIG. 2, a lower side in the drawing sheet is the patient 101 side. This view may be grasped as a perspective view showing the surface of an outer side of the generation part 7 (opposite side to the patient 101) . Further, this view may be grasped as a view showing the emission surface 7a of the generation part 7 in a plane perspective as well. The generation part 7, other than the configuration shown, may have for example a housing having a suitable shape covering the illustrated configuration from the opposite side to the patient 101 as well.

The generation part 7 for example has a plurality of flat-plate elements 11 which are linked with each other to configure substantially a dome shape. The flat-plate elements 11 are substantially configured in flat-plate shapes. From another viewpoint, the flat-plate elements 11 have substantially plane-shaped facing surfaces 11a (notation shown in FIG. 1) which face the patient 101 side. The plurality of facing surfaces 11a are linked with each other at their outer edges and configure the already explained recessed emission surface 7a. Accordingly, the emission surface 7a is not configured by a curved surface, but is configured by a combination of a plurality of planes.

The planar shapes of the flat-plate elements 11 and the dimensions of the planar shapes may be suitably set. For example, the plurality of flat-plate elements 11 may be given the same shapes and sizes as each other (example shown) or may include two or more types of flat-plate elements 11 which are different in shapes and/or sizes from each other. In the example shown, the planar shapes of the plurality of flat-plate elements 11 are made equilateral triangles having the same sizes as each other. However, the fact that a dome shape can be configured even by other polygons is apparent from regular polyhedrons (Platonic solids), semi-regular polyhedrons (Archimedean solids), platonic solids, and dome shapes which are realized in various technical fields.

As explained above, in the example shown, basically the planar shapes of the plurality of flat-plate elements 11 and their sizes are the same as each other. However, in a portion of the dome, flat-plate elements 11 having unique shapes may be provided as well. In the example shown, the deepest position (center part 7c) of the dome is made a region where no flat-plate elements 11 are arranged. In turn, the flat-plate elements 11 around the center part 7c are shaped as the planar shapes (triangles) of the other flat-plate elements 11 from which parts on the apex side are removed. In such a center part 7c, suitable electronic parts etc. may be arranged. For example, an ultrasonic sensor for detecting the position of the affected area 103 or a visual sensor which detects the position of a marker attached to the body surface of the patient 101 in order to show the position of the affected area 103 may be provided.

The plurality of flat-plate elements 11 may be linked with each other by a suitable method. In the example shown, the plurality of flat-plate elements 11 are indirectly linked with each other by being fixed to a frame 13. In more detail, the frame 13 extends along the outer edges (sides of the polygons) of the plurality of flat-plate elements 11 which are linked with each other. Further, each of the flat-plate elements 11 is fixed to a portion in the frame 13 which extend along its outer edge.

### (Flat-Plate Elements)

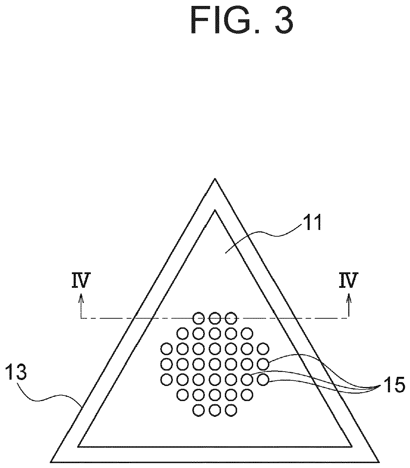

FIG. 3 is a view showing a portion corresponding to the one flat-plate element 11 in the generation part 7 and illustrating that one flat-plate element 11 in a plan view. In this view, in the frame 13, the portion corresponding to one flat-plate element 11 is shown.

The flat-plate element 11 has at least one (in the example shown, a plurality of) piezoelectric element 15. The piezoelectric elements 15 are portions which cause vibration for generating ultrasonic waves. The number, position, planar shape, and size etc. of the piezoelectric elements 15 may be suitably set.

In the example shown, the plurality of piezoelectric elements 15 are arranged along the plane direction of the flat-plate element 11 distributed with substantially uniform density. In more detail, the plurality of piezoelectric elements 15 are arranged with a constant pitches vertically and horizontally. However, the plurality of piezoelectric elements 15 may be offset by a half pitch between mutually neighboring columns, may be arranged along a plurality of concentric circles, may be radially arranged, or may be arranged with a density which is not uniform. Also, the correspondence of the directions of arrangement of the plurality of piezoelectric elements 15 and the directions of extension of the sides of the flat-plate element 11 may be suitably set.

The area of the region where the plurality of piezoelectric elements 15 are arranged (for example the smallest convex polygon into which the plurality of piezoelectric elements 15 are held) may be made for example 1/5 or more, 1/2 or more, 2/3 or more, or 4/5 or more of the area of the flat-plate element 11 (or the area surrounded by the frame 13 when viewed from the patient 101 side) . 4/5 or more may be grasped as a state where the plurality of piezoelectric elements 15 are arranged on the entire surface of the flat-plate element 11. If the plurality of piezoelectric elements 15 are not arranged over the entire surface of the flat-plate element 11, the region where the plurality of piezoelectric elements 15 are arranged may be positioned within a suitable range in the flat-plate element 11 such as the center side or outer edge side.

Further, in the example shown, the planar shapes of the piezoelectric elements 15 are made circular shapes. From another viewpoint, the planar shapes are line symmetrical or rotation symmetrical shapes. However, the planar shapes may be made elliptical or polygonal or other shapes. Further, the planar shapes may be asymmetrical shapes as well. In a case where the planar shapes of the piezoelectric elements 15 are not circular shapes, the relative orientations of the planar shapes of the piezoelectric elements 15 and the planar shape of the flat-plate element 11 may be suitably set.

### (Fixation Structure of Flat-Plate Elements and Frame)

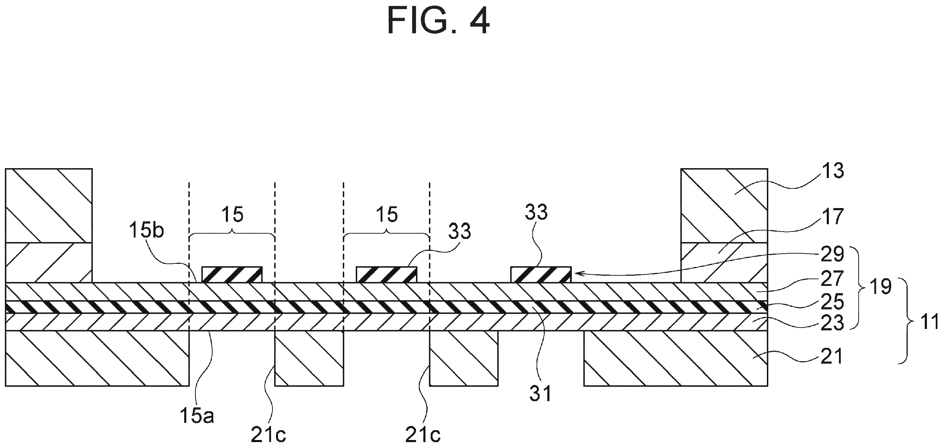

FIG. 4 is a cross-sectional view taken along the IV-IV line in FIG. 3. In this view, the lower part in the drawing sheet is the patient 101 side. In this view, for convenience of illustration, the thickness of part or all of the layer is exaggerated.

The flat-plate elements 11 may be positioned on the patient 101 side relative to the frame 13 or may be positioned on the opposite side to the patient 101. Simultaneously or alternatively, the frame 13 may have parts positioned between neighboring flat-plate elements 11, and the flat-plate elements 11 may be positioned on inner side in the plane direction from the frame 13. In the explanation of the present embodiment, an aspect where the flat-plate elements 11 are positioned on the patient 101 side relative to the frame 13 will be taken as an example.

The flat-plate elements 11 and the frame 13 are for example joined by a bonding material 17 interposed between the two. The bonding material 17 may be an organic material or may be an inorganic material. Further, the bonding material 17 may be an insulation material or may be a conductive material. For example, the bonding material 17 may be made a resin or metal.

Further, for example, the bonding material 17 may be for example made one which becomes an elastic body (for example elastic adhesive) after curing as well. Specifically, for example, the bonding material 17 may be silicone-based or urethane-based material. It may be a single-liquid type or may be a two-liquid type. Further, for example, the bonding material 17 forming the elastic body may be made with an elongation when it is torn in a tensile test after curing of 35% or more.

The flat-plate elements 11 (their side surfaces) may be separated from each other or may abut against each other. Further, in these cases, the flat-plate elements 11 may be directly fixed to each other (for example a bonding material 17 adhered to the two may be provided) or need not be fixed. In a case where the frame 13 has partition portions which are positioned between the side surfaces of the neighboring flat-plate elements 11, the side surfaces of the flat-plate elements 11 may be separated from the partition portions or may abut against the partition portions. In these cases, the side surfaces of the flat-plate elements 11 and the partition portions may be directly fixed or need not be fixed.

For fixation of the flat-plate elements 11 and the frame 13 and/or direct fixation of the flat-plate elements 11 with each other, in place of or addition to the method by the bonding material 17, another method may be utilized as well. As another method, for example, there can be mentioned engagement (interlocking), crimping, press-fitting, screwing, welding, fusing, etc.

### (Stacked Structure of Flat-Plate Elements)

The flat-plate element 11 has an element substrate 19 having piezoelectric elements 15 and a cavity member 21 which is superimposed on the element substrate 19 at the patient 101 side. The element substrate 19 vibrates by regions of the piezoelectric elements 15 flexurally deforming. This vibration is transferred to the fluid positioned in the substrate 19 on the patient 101 side whereby ultrasonic waves are generated. The cavity member 21 has a plurality of cavities 21c (openings, holes) which individually overlap the plurality of piezoelectric elements 15 in the element substrate 19. These cavities 21c contributes to for example raising the degree of directivity of the ultrasonic waves.

By provision of the plurality of cavities 21c and the later explained second electrodes 33 (individual electrodes) etc., the major surfaces (the broadest surfaces in the plate, front and back) of the flat-plate element 11 have relief shapes and are not planar. As understood from this, when referring to the flat-plate element 11 being flat plate shaped or to the flat-plate element 11 having a plane-shaped surface on the patient 101 side or opposite side to that, it need not be strictly a flat plate. For example, the flat-plate element 11 may be grasped to be a flat-plate shape by having a layer which has a constant thickness and forms planar surface (for example 23, 25, and 27 which will be explained later) as the principal component. Further, for example, the flat-plate element 11 may be grasped as a flat plate state when the top parts of the plurality of projecting portions (or the deepest parts in the recessed portions) fall in the same plane in each of the two major surfaces. Further, for example, the flat-plate element 11 may be grasped as a flat plate state when the arithmetic average roughness of the relief shapes of each major surface is 5% or less, 2% or less, or 1% or less of the circle equivalent diameter found from the area of the flat-plate element 11. Note that, in FIG. 4, the relief shapes of the major surfaces of the flat-plate element 11 are exaggerated.

### (Element Substrate)

The element substrate 19, for example, includes a vibration layer 23, first conductor layer 25, piezoelectric layer 27, and second conductor layer 29 in order from the patient 101 side (cavity member 21 side) . The first conductor layer 25 for example includes a first electrode 31. The second conductor layer 29 for example includes a plurality of second electrodes 33. The first electrode 31 and the second electrodes 33 sandwich the piezoelectric layer 27. By application of AC voltage to these pair of electrodes, in the regions forming the piezoelectric elements 15 in the element substrate 19, vibrations accompanied by flexural deformations are generated. Note that, other than the illustrated layers, the element substrate 19, for example, may include an insulation layer covering the second conductor layer 29 or other suitable layers. In the explanation of the present disclosure, a "layer" is a concept including a "plate".

In the element substrate 19, the regions which are grasped as the piezoelectric elements 15 may be suitably defined. In the explanation of the present embodiment, for convenience, in the element substrate 19, the regions overlapping the cavities 21c (more strictly speaking, the regions overlapping the element substrate 19 side opening surfaces of the cavities 21c) are defined as the piezoelectric elements 15. Each piezoelectric element 15 has a first surface 15a facing the cavity 21c side (patient 101 side) and a second surface 15b facing the opposite side to the cavity 21c. The first surface 15a is for example configured by the surface in the vibration layer 23 on the cavity member 21 side. The second surface 15b is for example configured by the surface of the second conductor layer 29 on the side opposite to the cavity member 21 and the regions in the surface of the piezoelectric layer 27 on the side opposite to the cavity member 21 which are exposed from the second conductor layer 29. Note that, for example, unlike the example shown, if an insulation layer covering the second conductor layer 29 is provided, the second surface 15b may be configured by this insulation layer.

### (Vibration Layer)

The vibration layer 23 for example spreads over substantially the entirety of the element substrate 19. In other words, the vibration layer 23 is formed in a solid pattern having a size covering the plurality of piezoelectric elements 15. The thickness of the vibration layer 23 is substantially constant. The vibration layer 23, for example, as will be explained later, contributes to generation of out-of-plane vibration by restricting the deformation in a plane direction of the piezoelectric layer 27. The vibration layer 23 may be integrally formed over its entirety or may be formed in a divided manner in a plane. The vibration layer 23 overlaps the cavity member 21. The vibration layer 23 may be grasped to be supported by the surrounding portions of the cavities 21c in the cavity member 21 as well.

The vibration layer 23 is for example formed by an insulation material or semiconductor material. The material of the vibration layer 23 may be an inorganic material or organic material. More specifically, for example, the material of the vibration layer 23 may be a piezoelectric substance which is the same as or different from the material of the piezoelectric layer 27 (explained later) . Further, for example, the material of the vibration layer 23 may be also made silicon (Si), silicon dioxide (SiO₂), silicon nitride (SiN), or sapphire (Al₂O₃). The vibration layer 23 may be configured by a plurality of layers made of mutually different materials stacked on each other as well. For example, the vibration layer 23 may be configured by a silicon layer and an SiO₂ layer superimposed on its upper surface and/or lower surface.

### (First Conductor Layer and First Electrode)

The first conductor layer 25, in the example shown, includes only the first electrode 31. The first electrode 31 is made a common electrode having a size large enough to cover the plurality of piezoelectric elements 15. The common electrode is for example formed in a solid pattern extending over substantially the entirety of the element substrate 19. The thickness of the common electrode is substantially constant. The first electrode 31 is for example electrically connected through a not shown via conductor passing through the piezoelectric layer 27 with a not shown wiring (for example cable) arranged on the opposite side to the bag 9 with respect to the flat-plate element 11.

The material of the first conductor layer 25 may be made for example a suitable metal. For example, use may be made of gold (Au), silver (Ag), palladium (Pd), platinum (Pt), aluminum (Al), nickel (Ni), copper (Cu), chromium (Cr), or an alloy including them. The first conductor layer 25 may be configured by a plurality of layers made of mutually different materials stacked on each other as well. Further, the material of the first conductor layer 25 may be one obtained by firing a conductive paste including a metal as explained before as well. That is, the material of the first conductor layer 25 may be one containing glass powder and/or ceramic powder or another additive (from another viewpoint, an inorganic insulation substance).

### (Piezoelectric Layer)

The piezoelectric layer 27 for example spreads over substantially the entirety of the element substrate 19. In other words, the piezoelectric layer 27 is formed in a solid pattern having a size covering the plurality of piezoelectric elements 15. The thickness of the piezoelectric layer 27 is substantially constant.

The material of the piezoelectric layer 27 may be monocrystalline, may be polycrystalline, may be an inorganic material, may be an organic material, may be or may not be a ferroelectric substance, or may be or may not be a pyroelectric body. As the inorganic material, for example, there can be mentioned a lead zirconate titanate-based material and a lead-free inorganic piezoelectric material. As the lead-free inorganic piezoelectric material, for example, there can be mentioned a perovskite type compound material. As the organic material, for example, there can be mentioned PVDF (polyvinylidene fluoride).

Further, the material of the piezoelectric layer 27 may be made for example a piezoelectric ceramic plate (from another viewpoint, a sintered body) or may be made a piezoelectric thin film. The piezoelectric ceramic plate is a plate-shaped inorganic polycrystal substance configured by a plurality of crystal grains (and crystal grain boundaries) having piezoelectric characteristics. The crystal grains configuring the piezoelectric ceramic plate usually have small aspect ratios, and are isotropically distributed. The piezoelectric thin film is an inorganic single crystal, an inorganic polycrystal substance, or an organic material (polymer), which has a thin film shape and a piezoelectric characteristic. The piezoelectric thin film of the polycrystal substance is usually configured by a columnar crystal extending in the thickness direction. The piezoelectric thin film usually has a high orientation and thereby has a high piezoelectric characteristic.

The piezoelectric layer 27, for example, in at least the regions configuring the piezoelectric elements 15, has a polarization axis (also referred to as an electrical axis or X-axis in a single crystal) which becomes substantially parallel to the thickness direction of the piezoelectric layer 27 (facing direction of the first electrode 31 and the second electrodes 33) . Note that, in the piezoelectric layer 27, the regions other than the regions configuring the piezoelectric elements 15 may be polarized or may not be polarized. Further, in a case where they are polarized, they may be polarized in the same direction as the regions configuring the piezoelectric elements 15 or may be polarized in a different direction.

### (Second Conductor Layer and Second Electrodes)

The second conductor layer 29, for example, other than the already explained plurality of second electrodes 33, may have not shown wirings which are connected to the plurality of second electrodes 33. The plurality of second electrodes 33 are for example electrically connected through not shown wirings included in the second conductor layer 29 with not shown other wirings (for example cables) arranged on the opposite side to the bag 9 with respect to the flat-plate element 11.

The plurality of second electrodes 33 are for example made individual electrodes provided for every piezoelectric element 15. The "individual electrodes" referred to here mean that the plurality of electrodes are separated from each other. They need not be able to be given mutually different potentials . For example, two or more second electrodes 33 (for example, all second electrodes 33 in one flat-plate element 11) may be connected to each other. The connection may be for example made by not shown wirings provided in the second conductor layer 29 or may be carried out by other means (for example bonding wires). Note that, the plurality of second electrodes 33 may be able to be given mutually different potentials individually or for each group including two or more second electrodes 33.

The planar shapes and sizes of the second electrodes 33 may be made suitable ones. For example, the planar shapes of the second electrodes 33 may be similar shapes or resembling shapes to the planar shapes of the piezoelectric elements 15 (shapes of openings of the cavities 21c) or may be different shapes from the later shapes. Further, for example, when viewed on a plane, the outer edges of the second electrodes 33 may in their entirety be positioned at the inner sides relative to the edge parts of the openings of the cavities 21c, may in their entirety substantially coincide with the edge parts, may in their entirety be positioned at the outer sides relative to the edge parts, or may only partially coincide or be positioned at the inner sides. In the present embodiment, the second electrodes 33 are circular shapes positioned at the inner sides from the edge parts of the openings of the circular cavities 21c.

The material of the second conductor layer 29 may be the same as or may be different from the material of the first conductor layer 25. Further, for any case, the already given explanation of the material of the first conductor layer 25 may be employed for the explanation of the material of the second conductor layer 29.

### (Operations of Piezoelectric Elements 15)

If an electric field is supplied by the first electrode 31 and the second electrodes 33 to the piezoelectric layer 27 sandwiched between them in the same orientation as the orientation of polarization, the piezoelectric layer 27 contracts in the plane direction. This contraction is restricted by the vibration layer 23, therefore the piezoelectric elements 15 flex (displace) to the cavity 21c side like a bimetal. Conversely, if an electric field is supplied in an inverse orientation to the orientation of the polarization, the piezoelectric elements 15 flex to the opposite side to the cavities 21c.

Due to displacements of the piezoelectric elements 15 explained above, pressure waves are formed in the media (for example fluid) surrounding the piezoelectric elements 15. Further, by an electrical signal (driving signal) changing in voltage with a predetermined waveform being input to the first electrode 31 and second electrodes 33, ultrasonic waves reflecting the waveform (from another viewpoint, the frequency and amplitude) of that electrical signal are generated.

The vibration of the flexural deformation described above, in other words, is out-of-plane vibration (bending vibration) of a primary mode in which, in the piezoelectric elements 15, the center when viewed on a plane becomes the antinode of the vibration and the outer edge (for example the vicinity of the edge part of the cavity 21c) becomes the node of the vibration. Concerning this vibration, the piezoelectric elements 15 are for example configured so that the resonance frequency is positioned in the frequency band of the ultrasonic waves. The resonance frequency is for example set by selection of the material of the layer configuring the piezoelectric elements 15 (from another viewpoint, selection of Young's modulus and density) and setting of the diameters of the piezoelectric elements 15 and the thickness of each layer (from another viewpoint, setting of the mass and bending rigidity) etc. The influence of the fluid surrounding the piezoelectric elements 15 and the influence of the rigidity etc. of the portions supporting the piezoelectric elements 15 (for example the cavity member 21) may be considered as well.

The electrical signal may be for example a signal repeatedly applying voltage for making the piezoelectric elements 15 displace to the cavity 21c side and applying voltage for making the piezoelectric elements 15 displace to the opposite side to the cavities 21c. That is, the electrical signal may be a signal inverting in polarity (positive/negative) (orientations of the voltages (electric fields) alternately switching with each other in the direction of the polarization axis). Further, for example, the electrical signal may be a signal repeatedly only applying voltage for making the piezoelectric elements 15 displace to the cavity 21c side or only applying voltage for making the piezoelectric elements 15 displace to the opposite side to the cavities 21c. In this case, ultrasonic waves are generated by repetition of flexing and elimination of flexing by a restoring force.

### (Cavity Member)

The cavity member 21 is for example a member with a constant thickness which has a size covering the plurality of piezoelectric elements 15 when considered ignoring the cavities 21c. The material of the cavity member 21 may be any material. For example, it may be an insulation material, may be a semiconductor material, may be a conductive material, may be an inorganic material, may be an organic material, may be a piezoelectric substance, or may be the same as the material of any layer in the element substrate 19. Specifically, as the material of the cavity member 21, there can be mentioned a metal, resin, and ceramic. Further, the cavity member 21 may be configured by a plurality of materials or plurality of layers. For example, the cavity member 21 may be configured by an insulation layer formed on a metal layer (including a metal plate) superimposed on the element substrate 19 or may be configured by a glass epoxy resin by impregnation of an epoxy resin into glass fiber.

The shapes of the cavities 21c may be suitably set. For example, the shapes of the cavities 21c may be shapes constant in transverse cross-sections (cross-sections parallel to the element substrate 19) regardless of the positions in the penetration directions of the cavities 21c (example shown) or may be shapes having a tapered surfaces increasing in diameter or decreasing in diameter the further to the element substrate 19 side. In the explanation of the present embodiment, the regions of the element substrate 19 which overlap the cavities 21c are deemed piezoelectric elements 15, therefore the explanation already given for the planar shapes of the piezoelectric elements 15 may be employed for the explanation of the shapes of the transverse cross-sections of the cavities 21c.

The depths of the cavities 21c (lengths in the penetration direction, from another viewpoint, the thickness of the cavity member 21) may be suitably set. For example, the depths of the cavities 21c may be made 1/20 or more, 1/10 or more, 1/2 or more, or 1 time or more of the diameters of the cavities 21c (when not circular shapes, for example, circle equivalent diameters) or may be made 10 times or less, 5 times or less, 1 time or less, 1/2 or less, or 1/10 or less. The above lower limits and upper limits may be suitably combined unless they are contradictory.

### (One Example of Dimensions etc.)

As already explained, in the emission device 3, the frequency of the ultrasonic waves and dimensions etc. of the parts of the emission device 3 may be suitably set. Below, one example will be explained. The frequency of the ultrasonic waves generated by the emission device 3 may be made 0.5 MHz to 2 MHz. The diameter of the generation part 7 (diameter of the opening surface of the dome) may be made 50 mm to 200 mm. The circle equivalent diameters of the flat-plate elements 11 or single sides of the triangular flat-plate elements 11 may be made 5 mm to 20 mm. The circle equivalent diameters of the piezoelectric elements 15 may be made 0.2 mm to 2 mm. The thickness of the element substrate 19 may be made 50 pm to 200 pm. The respective thicknesses of the thickness of the vibration layer 23 and the thickness of the piezoelectric layer 27 may be made 20 pm to 100 pm within a range not contradicting the thickness of the element substrate 19 explained before. The respective thicknesses of the first conductor layer 25 (first electrode 31) and second conductor layer 29 (second electrodes 33) may be made 0.05 pm to 5 pm.

### (bag)

Returning to FIG. 1, the shape, size, and material of the bag 9 may be suitably set. For example, the shape of the bag 9 may be made a sphere or other shape that swells outward as a whole. Further, for example, in a case where the emission device 3 is aimed at a specific portion of the human body, it may have such a shape that has a projecting portion and/or recessed portion matching with the recessed portion and/or projecting portion of the specific portion.

The material of the bag 9 has at least the property of not allowing the liquid LQ to pass through it (so-called water barrier property) and flexibility. Further, the material of the bag 9 may be an elastic body as well. For example, as the material of the bag 9, use may be made of a thermosetting elastomer (so-called rubber), thermoplastic elastomer (elastomer in a narrow sense), and a resin which does not contain these elastomers (resin in a narrow sense, however, one having flexibility). As the thermosetting elastomer, there can be mentioned vulcanized rubber (rubber in a narrow sense) and thermosetting resin-based elastomer.

In the bag 9, as already explained, at least the liquid LQ is sealed in at the time of use of the ultrasound apparatus 1. The bag 9 (emission device 3) may be for example one in which the liquid LQ has been sealed in at the stage of distribution or may be one in which the liquid LQ is sealed in at the time of use. Further, the bag 9 (emission device 3) may be for example one not having a port which can be opened or closed for supplying (and/or discharging) the liquid LQ to/from the interior of the bag 9 or may be one having such a port. For the opening/closing structure of the port, known various ones may be utilized.

The bag 9 has an opening 9a on the generation part 7 side. Further, in the generation part 7, part including the emission surface 7a is in contact with the liquid LQ in the bag 9 through the opening 9a. On the other hand, the part of the generation part 7 which includes the surface on the opposite side to the emission surface 7a does not contact the liquid LQ, but contacts the gas (for example air) surrounding the emission device 3.

The structure for reducing leakage of the liquid LQ from a gap between the edge parts of the opening 9a and the generation part 7 may be made various known sealing structures. For example, the bag 9 and the generation part 7 may be bonded by an adhesive or may be welded and/or fused together by melting of at least one of the bag 9 and the generation part 7 or otherwise closely bonded. Further, for example, when the bag 9 is made of an elastic body, in a state where the inner circumferential surface near the opening 9a in the bag 9 is pushed against the outer circumferential surface of the generation part 7, the vicinity of the opening 9a in the bag 9 may be fastened from the outside by a string or ring-shaped fastener. Further, for example, in a state where the inner circumferential surface in the vicinity of the opening 9a of the bag 9 is pushed against the outer circumferential surface of the generation part 7, a ring-shaped packing may be made to abut against the vicinity of the opening 9a of the bag 9 from the outside and the packing fastened from the outside by a string or ring-shaped fastener. Further, for example, a ring-shaped member (rigid body) which is not flexible configuring the vicinity of the opening 9a may be provided in the bag 9, a female screw may be provided on an inner side of this ring-shaped member, a male screw may be provided on the outer surface of the generation part 7, and the two may be screwed together. Further, the packing may be suitably arranged in this screwing structure as well.

In FIG. 2, only the frame 13 and flat-plate elements 11 were shown as the configurations of the generation part 7. The bag 9 may be attached to for example the part in the frame 13 which configures the opening surface on the patient 101 side. Further, for example, the generation part 7 may have a not shown attachment member which is closely fastened to the part of the frame 13 configuring the above opening surface and the bag 9 may be attached to the attachment member. The attachment member may be for example a frame-shaped member wider than the parts of the frame 13 or may be a housing shaped member covering the side of the frame 13 opposite to the patient 101. In the schematic view in FIG. 1, the connection position of the generation part 7 and the opening 9a is positioned between the emission surface 7a and its back face. However, the connection position may be positioned at the side closer to the patient 101 than the emission surface 7a or may be positioned at the opposite side to the patient 101 more than the back face of the emission surface 7a. Further, the bag 9 may circle around the generation part 7 to the opposite side to the patient 101 or the liquid LQ may circle around the generation part 7 to the opposite side to the patient 101. In that case, for example, a structure providing a cover surrounding the second surfaces 15b of the piezoelectric elements 15 and isolating the second surfaces 15b of the piezoelectric elements 15 from the liquid LQ may be employed.

### (Fluid around Piezoelectric Elements)

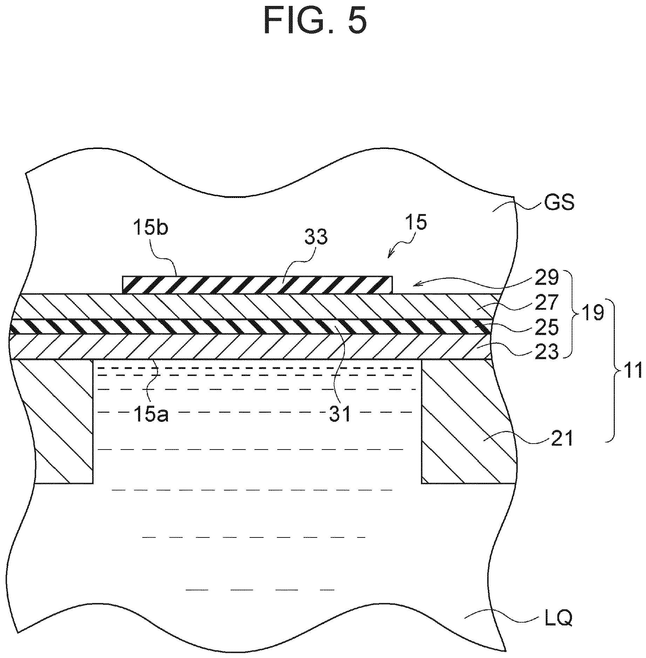

FIG. 5 is a view showing a portion in FIG. 4 in an enlarged manner. As understood from the above explanation, in the piezoelectric elements 15, the first surface 15a on the patient 101 side is in contact with the liquid LQ, and the second surface 15b on the opposite side is in contact with a gas GS (for example air).

### (Apparatus Body)

Returning to FIG. 1, the apparatus body 5 for example has a driving control part 41 which inputs a driving signal to the emission device 3, a movement part 43 moving the emission device 3, an input part 45 which receives an input operation by a user, and an output part 47 which shows information to the user.

The driving control part 41 is for example connected through a cable 49 with the first electrode 31 and plurality of second electrodes 33 in the generation part 7. The driving control part 41 has a driving part 51 which inputs driving signals to the first electrode 31 and second electrodes 33 and a control part 53 which controls the driving part 51.

The driving part 51 for example converts electric power from a commercial power supply or the like to an AC power having a waveform (for example frequency and voltage (amplitude)) designated by the control part 53 and inputs the result to the first electrode 31 and second electrodes 33. The driving signal is AC power which has a frequency substantially equal to the frequency of the ultrasonic waves to be emitted and has a voltage corresponding to the amplitude of the intended ultrasonic waves. The driving signal may be made a rectangular wave (pulse), sine wave, triangular wave, or sawtooth wave or another suitable shape.

The control part 53, although not particularly shown, is configured including a computer which includes a CPU (central processing unit), ROM (read only memory), RAM (random access memory), external storage device, etc. The CPU runs a program stored in the ROM and/or external storage device to construct function parts performing various types of control. The control part 53, for example, sets the waveform (for example frequency and voltage (amplitude)) of the driving signal output by the driving part 51 based on a signal from the input part 45. Further, the control part 53 controls the start and stopping of the output of the driving signal from the driving part 51.

The movement part 43, for example, although not particularly shown, is configured including a holding mechanism which holds the emission device 3 and a driving source (for example motor) which gives drive power to the holding mechanism for moving the emission device 3. Such a movement part 43, for example, may be configured the same as an articular robot, scalar robot, or orthogonal robot. The movement part 43 makes the emission device 3 relatively move with respect to the patient 101 based on a control command from the control part 53. This relative movement may include for example movement of making the emission device 3 approach the patient 101 and/or movement for positioning to set a focal point of the ultrasonic waves at the affected area 103. The control part 53 controls the movement part 43 based on a signal from the input part 45 and/or based on a signal from a not shown sensor for identifying the position of the affected area 103 etc. Note that, the movement part 43 need not be provided or the driving source in the movement part 43 need not be provided. The emission device 3 may be carried and positioned manually.

The input part 45 is for example configured including a keyboard, mouse, mechanical switches, and/or a touch panel. The input part 45, for example, receives an operation for setting the frequency and amplitude of the ultrasonic waves emitted from the emission device 3 and an operation for instructing the start and stopping of emission of the ultrasonic waves. The output part 47 is for example configured including a display device and/or speaker. The output part 47, for example, shows the information of the frequency and amplitude of the ultrasonic waves set at the present moment etc.

As explained above, in the present embodiment, the ultrasound emission device 3 has the generation part 7 generating the ultrasonic waves which are emitted to the object (patient 101) side and the bag 9 in which the liquid LQ is sealed so that the liquid LQ is positioned on the patient 101 side relative to the generation part 7. The generation part 7 has the piezoelectric elements 15 which generate vibrations generating the ultrasonic waves. The piezoelectric elements 15 have the first surfaces 15a facing the patient 101 side and the second surfaces 15b facing the opposite side to the patient 101 side. Further, between the first surfaces 15a and the second surfaces 15b, the piezoelectric elements 15 have piezoelectric layers 27 extending along the above surfaces and pairs of electrodes (first electrode 31 and second electrodes 33) overlapping the two surfaces of the piezoelectric layers 27. Further, the piezoelectric elements 15 are configured to flexurally deform so that the first surfaces 15a and the second surfaces 15b flex together to the same sides as each other by application of voltage to the first electrode 31 and second electrodes 33. The first surfaces 15a are exposed inside the bag 9 so as to contact the liquid LQ. The second surfaces 15b are isolated from the liquid LQ and are in contact with the gas GS.

Accordingly, for example, by the first surfaces 15a of the piezoelectric elements 15 directly contacting the liquid LQ, the vibrations generated in the piezoelectric elements 15 are directly transferred to the liquid LQ. As a result, the ultrasonic waves can be efficiently emitted to the patient 101 through the liquid LQ. Specifically, in a case where a solid is interposed between the first surfaces 15a and the liquid LQ, the ultrasonic waves end up being reflected at the interfaces between the first surfaces 15a and the solid and/or the interface between the solid and the liquid LQ, or the ultrasonic waves end up being leaked through the solid in a plane direction of the first surfaces 15a. In the present embodiment, however, such reflection and/or leakage of the ultrasonic waves is reduced. On the other hand, the second surfaces 15b of the piezoelectric elements 15b are in contact with the gas GS. Therefore, compared with an aspect where the second surfaces 15b are in contact with a solid, the force acting to suppress the flexural deformation of the second surfaces 15b becomes small, therefore vibrations can be efficiently generated. Further, the second surfaces 15b of the piezoelectric elements 15 are in contact with the gas GS. Therefore, compared with an aspect where the second surfaces 15b contact the liquid, the amount of emission of the generated ultrasonic waves toward the patient 101 side can be increased.

Further, in the present embodiment, the generation part 7 has the cavity member 21 which is positioned on the patient 101 side relative to the piezoelectric elements 15. The cavity member 21 has the cavities 21c which run from the first surface 15a side to the patient 101 side and expose the first surfaces 15a to the interior of the bag 9.

In this case, for example, waves in directions greatly inclined from the first surfaces 15a relative to the normal lines of the first surfaces 15a are blocked (for example reflected) by the inner surfaces of the cavities 21c. Accordingly, the degree of the directivity of the ultrasonic waves emitted from the flat-plate elements 11 can be raised. As a result, for example, the ultrasonic waves are effectively emitted to the intended position.

Further, in the present embodiment, the generation part 7 has the element substrate 19 including the plurality of piezoelectric elements 15. The cavity member 21 has the plurality of cavities 21c which are superposed on the element substrate 19 from the patient 101 side and individually overlap the plurality of piezoelectric elements 15.

In this case, for example, the cavities 21c can lower the probability of the ultrasonic waves from the plurality of piezoelectric elements 15 interfering with each other. As a result, for example, the probability of generation of an ultrasonic component having an unwanted amplitude, frequency, or direction is lowered. In turn, for example, the intended therapeutic effect is easily obtained. Further, the cavity member 21 is superposed on the element substrate 19. Therefore, for example, the cavity member 21 contributes to suppression of vibrations around the regions (piezoelectric elements 15) in the element substrate 19 which overlap the cavities 21c and raising the resonance frequencies of the piezoelectric elements 15. As a result, it is made easier to generate sound waves with relatively high frequencies, that is, ultrasonic waves, and/or generate waves having relatively high frequencies in the frequency band of the ultrasonic waves.

Further, in the present embodiment, the generation part 7 has the plurality of flat-plate elements 11 of flat plate shapes. Each flat-plate element 11 has at least one piezoelectric element 15 so that the first surface 15a and the second surface 15b are along a plane direction of the flat-plate element 11. The plurality of flat-plate elements 11 are linked with each other at their outer edges and configure a recessed surface (emission surface 7a) which is recessed at the patient 101 side.

In this case, for example, the generation part 7 can make the ultrasonic waves focus at the curvature center side of the recessed surface. Further, the flat-plate elements 11 can be prepared by the same method as a method of preparing a usual circuit board etc. As a result, the manufacturing costs are reduced. Further, for example, it is made easy to equally emit the focused ultrasonic waves to a relatively broad emission region (affected area 103) .

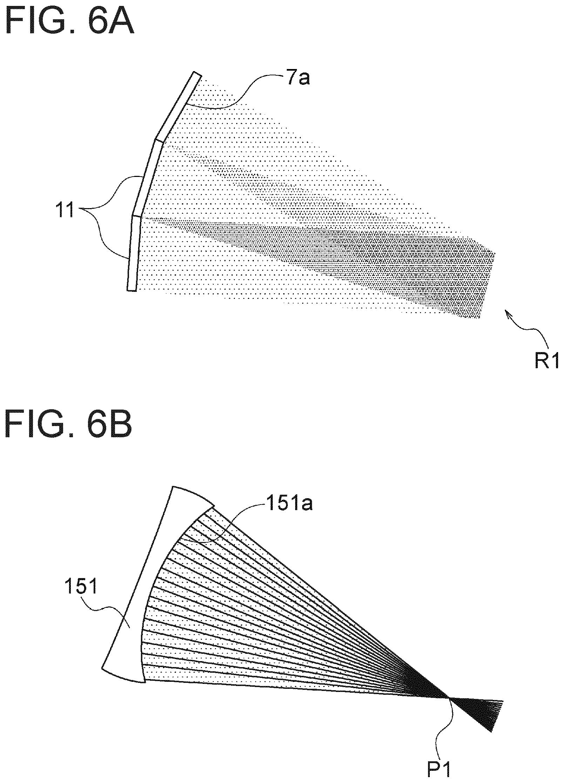

FIG. 6A and FIG. 6B are schematic views for explaining the effect of emission to the above broad emission region. Specifically, FIG. 6A and FIG. 6B schematically show states of focus of the ultrasonic waves in the present embodiment and a comparative example.

First, as a comparative example, as shown in FIG. 6B, consider an element 151 having a curved surface-shaped emission surface 151a. The emission surface 151a is for example configured by a single plate-shaped lens member. Elements (not shown) corresponding to the plurality of piezoelectric elements 15 are arranged behind the lens member. Further, the lens member makes the ultrasonic waves generated by the plurality of piezoelectric elements 15 focus to a focal point PI. The focal point P1 is in theory a point, therefore the ultrasonic waves are focused to a relatively narrow range.

On the other hands, as shown in FIG. 6A, the ultrasonic waves emitted from the flat-plate elements 11 are emitted to a focusing region R1 with widths (widths of beams) at emission from the flat-plate elements 11 as they are. Further, the ultrasonic waves of the plurality of flat-plate elements 11 are focused. Accordingly, the focusing region R1 theoretically becomes a region having the same extent of width as the widths of the ultrasonic waves emitted from the flat-plate elements 11. Further, within the width of this focusing region R1, the strengths of the ultrasonic waves are substantially equal. Depending on the size of the affected area 103 and type of disease or the like, the formation of such a focusing region R1 is efficient and/or safe.

Further, in the present embodiment, each of the plurality of flat-plate elements 11 has a plurality of piezoelectric elements 15 which are arranged distributed along the plane direction of each flat-plate element 11.

In this case, it is easy to make the widths of the ultrasonic waves (widths of the beams) emitted from the flat-plate elements 11 substantially large, therefore the effects explained with reference to FIG. 6A are easily obtained. Further, the resonance frequencies of flexural vibrations of the piezoelectric elements 15 can be made closer to the frequencies of the ultrasonic waves which are generated, therefore the ultrasonic waves can be efficiently generated. In this way, by each flat-plate element 11 having the plurality of piezoelectric elements 15, ultrasonic waves having broad widths of beams can be efficiently emitted.

Further, in the present embodiment, the generation part 7 has the frame 13 to which the outer edges of the plurality of flat-plate elements 11 are fixed. An elastic body (bonding material 17 made of an elastic adhesive) is interposed between the plurality of flat-plate elements 11 and the frame 13.

In this case, for example, the vibrations transferred from the piezoelectric elements 15 to the frame 13 can be reduced. As a result, unwanted vibrations of the entirety of the generation part 7 are reduced. In turn, for example, the probability of unpleasant vibration which is not useful for medical treatment being transferred to the patient 101 or abnormal noise in an audible range being generated in the emission device 3 can be lowered.

### (Modifications)

Below, modifications will be explained. In the explanations of the modifications, basically only different portions from the embodiment will be explained. The matters which are not particularly explained may be made the same as those in the embodiment. Further, for convenience of explanations, configurations corresponding to the configurations in the embodiment will sometimes be assigned the same notations even if there is a difference.

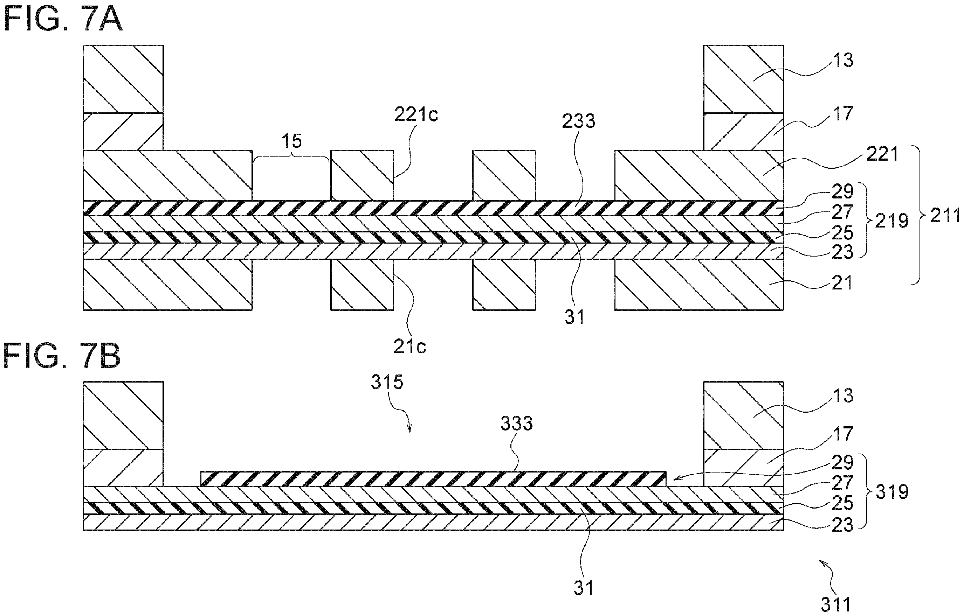

FIG. 7A is a view showing the configuration of a flat-plate element 211 according to a modification and corresponds to FIG. 4.

In the explanation of the embodiment, it was stated that the plurality of second electrodes 33 (individual electrodes) may be made the same potentials as each other. Accordingly, as shown in FIG. 7A, the second electrode 233, in the same way as the first electrode 31, may be made a common electrode as well. That is, the second electrode 233 may be made a solid pattern extending over the plurality of piezoelectric elements 15 as well. The second electrode 233 for example has an equal size to the element substrate 219. Although not particularly shown, the first electrode may be made individual electrodes and be combined with the plurality of second electrodes 33 in FIG. 4 or the second electrode 233 in FIG. 7A.

Further, in the modification shown in FIG. 7A, a cavity member 221 is also provided on the opposite side to the patient 101. The cavity member 221 has the same configuration as that of the cavity member 21 and has cavities 211c overlapping the piezoelectric elements 15. In a case where such a cavity member 221 is provided, the cavity member 21 on the patient 101 side may be provided (example shown) or may be removed. Further, this cavity member 221, in place of the second electrode 233, may be combined with the second electrodes 33 (individual electrodes) in the embodiment as well. Further, the cavity member 221 may be bonded through the bonding material 17 with the frame 13 as well.

FIG. 7B is a view showing the configuration of a flat-plate element 311 according to another modification and corresponds to FIG. 4.

As shown in this view, the flat-plate element 311 may have only a single piezoelectric element 315 as well. From another viewpoint, the first electrode 31 and second electrode 333 need not be grasped by the concepts of individual electrodes and a common electrode either. Note that, in the example shown, the area of the second electrode 333 is made smaller than the area of the flat-plate element 319. However, in the same way as the modification in FIG. 7A, the area of the second electrode 333 may be made equal to the size of the flat-plate element 319 as well.

Further, in this modification, the cavity member 21 is not provided. In other words, the flat-plate element 311 is configured by only the element substrate 319. In the example shown, the piezoelectric element 315 for example generates vibration of flexural deformation using the fixed portion with the frame 13 as the node of vibration. Naturally, even in a case where the flat-plate element 311 has only one piezoelectric element 315, a cavity member having one cavity overlapping this one piezoelectric element 315 may be provided as well.

In the above embodiment and modifications, the patient 101 or affected area 103 is one example of the object. The emission surface 7a is one example of the recessed surface. The bonding material 17 is one example of the elastic body.

The technique according to the present disclosure is not limited to the above embodiment and modifications and may be executed in various ways.

For example, the ultrasound emission device and ultrasound apparatus are not limited to ones used for medical treatment. For example, they may be utilized in ultrasound diagnosis apparatuses obtaining cross-sectional two-dimensional images of a patient as well. Further, for example, they are not limited to the medical field and may be utilized in various fields for imparting energy and/or measuring distance.

The elastic body interposed between the flat-plate elements and the frame is not limited to an elastic adhesive. For example, each of the flat-plate elements and frame may be fixed by an adhesive or the like to a not elastic adhesive elastic body which is interposed between the flat-plate elements and the frame.

From the technique according to the present disclosure, the following concept which is not predicated on a bag can be extracted.

### (Concept 1)

An ultrasound emission device including a generation part generating ultrasonic waves which are emitted to an object side, wherein
the generation part includes flat-plate shaped plurality of flat-plate elements facing the object side,
the plurality of flat-plate elements are linked with each other at their outer edges and configure a recessed surface making the object side recessed, and
each of the plurality of flat-plate elements includes at least one piezoelectric element which vibrates in a direction which the flat-plate element faces and generates an ultrasonic wave.

### Reference Signs List

1... ultrasound apparatus, 3... ultrasound emission device, 7... generation part, 9...bag, 15... piezoelectric element, 15a... first surface, 15b... second surface, 17... piezoelectric layer, 31... first electrode (electrode), 33... second electrode (electrode), 101... patient (object), LQ... liquid, and GS... gas.

## Claims

1. An ultrasound emission device comprising:
a generation part generating an ultrasonic wave which is emitted to an object side; and
a bag in which a liquid is sealed so that the liquid is located on the object side relative to the generation part, wherein
the generation part comprises a piezoelectric element which generates vibration generating the ultrasonic wave,
the piezoelectric element
comprises
a first surface facing the object side, and
a second surface facing an opposite side to the object side,
comprises, between the first surface and the second surface,
a piezoelectric layer lying along the first surface and the second surface, and
a pair of electrodes which overlap two surfaces of the piezoelectric layer, and
is configured so as to flexurally deform so that the first surface and the second surface flex to a same side as each other by application of voltage to the pair of electrodes,
the first surface is exposed to an inside of the bag so as to contact the liquid, and
the second surface is isolated from the liquid and is in contact with a gas.

2. The ultrasound emission device according to claim 1, wherein
the generation part comprises a cavity member which is located on the object side relative to the piezoelectric element, and
the cavity member comprises a cavity which goes from a side where the first surface is located to the object side and exposes the first surface to the inside of the bag.

3. The ultrasound emission device according to claim 2, wherein
the generation part comprises an element substrate that includes a plurality of the piezoelectric elements, and
the cavity member comprises a plurality of the cavities which are superposed on the element substrate from the object side and individually overlap the plurality of piezoelectric elements.

4. The ultrasound emission device according to any one of claims 1 to 3, wherein
the generation part comprises a plurality of flat-plate elements of flat plate shapes,
each of the plurality of flat-plate elements comprises at least one of the piezoelectric element so that the first surface and the second surface are along a plane direction of each flat-plate element, and
the plurality of flat-plate elements are linked with each other at their outer edges and configure a recessed surface making the object side recessed.

5. The ultrasound emission device according to claim 4, wherein each of the plurality of flat-plate elements comprises a plurality of the piezoelectric elements which are arranged distributed along the plane direction of each of the flat-plate elements.

6. The ultrasound emission device according to claim 4 or 5, wherein
the generation part comprises a frame to which the outer edges of the plurality of flat-plate elements are fixed, and
one or more elastic bodies are interposed between the plurality of flat-plate elements and the frame.

7. An ultrasound apparatus comprising:
the ultrasound emission device according to any one of claims 1 to 6, and
a driving control part which supplies an AC voltage having a frequency in a frequency band of ultrasound to the pair of electrodes.
